(19) Europäisches Patentamt / European Patent Office / Office européen des brevets

(11) **EP 2 533 697 B1**

(12) **EUROPEAN PATENT SPECIFICATION**

(45) Date of publication and mention
of the grant of the patent:
**28.09.2016 Bulletin 2016/39**

(21) Application number: **11708332.9**

(22) Date of filing: **01.02.2011**

(51) Int Cl.:
*A61B 8/08* (2006.01)     *A61B 5/00* (2006.01)
*G01N 21/17* (2006.01)    *G01N 29/24* (2006.01)
*G01N 29/07* (2006.01)

(86) International application number:
**PCT/JP2011/000554**

(87) International publication number:
**WO 2011/096198 (11.08.2011 Gazette 2011/32)**

(54) **PHOTOACOUSTIC IMAGING APPARATUS AND PHOTOACOUSTIC IMAGING METHOD**

PHOTOAKUSTISCHE BILDGEBUNGSVORRICHTUNG UND PHOTOAKUSTISCHES
BILDGEBUNGSVERFAHREN

APPAREIL D'IMAGERIE PHOTOACOUSTIQUE ET PROCÉDÉ D'IMAGERIE PHOTOACOUSTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.02.2010 JP 2010025864**

(43) Date of publication of application:
**19.12.2012 Bulletin 2012/51**

(73) Proprietor: **Canon Kabushiki Kaisha
Tokyo 146-8501 (JP)**

(72) Inventors:
• **FUKUTANI, Kazuhiko
Tokyo 146-8501 (JP)**
• **ASAO, Yasufumi
Tokyo 146-8501 (JP)**

(74) Representative: **TBK
Bavariaring 4-6
80336 München (DE)**

(56) References cited:
**EP-A1- 1 935 346     EP-A2- 1 700 563**

## Description

## Technical Field

The present invention relates to a photoacoustic imaging apparatus and a photoacoustic imaging method in which an acoustic wave that is generated from an inner portion of a sample by irradiating the sample with light is detected, and a detection signal thereof is processed to obtain image data of the inner portion of the sample.

## Background Art

In the medical field, research in optical imaging apparatuses that irradiate a living body with light from a light source, such as a laser, and that performs imaging on information of an inner portion of the living body obtained on the basis of incident light is being positively conducted. Photoacoustic tomography (PAT) is one example of optical imaging technology. In the photoacoustic tomography, a living body is irradiated with pulsed light generated from a light source, and an acoustic wave (typically, an ultrasonic wave) generated from living tissues that have absorbed energy of the pulsed light propagated through/scattered in the living body is detected. That is, by making use of the difference between light energy absorptance of a sample (such as a tumor) and those of tissues other than the sample, a transducer receives an elastic wave that is generated when the sample absorbs the light energy with which the sample is irradiated and expands instantaneously. By mathematically analyzing a detection signal, an optical characteristics distribution, in particular, absorption coefficient distribution of the inner portion of the living body can be obtained. These items of information can be used in quantitative measurements of specific substances in the sample, such as hemoglobin and glucose contained in blood. In recent years, using the photoacoustic tomography, preclinical research in which imaging is performed on blood vessels of small animals and clinical research applying this principle to diagnosis of, for example, breast cancer are positively being conducted.

In the photoacoustic tomography, ordinarily, in the process of mathematically analyzing the detection signal (reconstructing the image), the average sound speed in the inner portion of the sample is used for calculation. In general, the average sound speed in the inner portion of the sample used in reconstructing the image is set on the basis of, for example, experimental values and reference values. However, since the sound speeds at samples depend upon, for example, a holding method of the samples, if the average sound speed used in reconstructing the image differs from an actual sound speed in the inner portion of the sample, an error occurs in the calculation for reconstructing the image, thereby considerably reducing the resolution of the obtained image. This is because a generally used image reconstruction theory assumes that the velocity of an acoustic wave that propagates in an imaging area is constant. This is a problem based on the principle of image reconstruction theory of photoacoustic tomography.

A document that discusses a technology of determining the sound speed in a sample using PAT is PTL 1. In PTL 1, an acoustic wave generated by irradiating a very small optical absorber (an acoustic generator) with light without a sample and an acoustic wave generated by irradiating a sample with light are obtained separately. The very small optical absorber is installed outside the location where the sample is installed. By comparing signals thereof with each other and analyzing them, a sound speed distribution of the inner portion of the sample can be calculated. It is known that the sound speed in a cancerous tissue differs locally from those in the vicinity thereof. By using an image obtained by this method, it is possible to diagnose the sample.

## Citation List

## Patent Literature

PTL 1: European Patent No. 1935346

## Summary of Invention

However, in the PTL 1, the purpose is to determine the sound speed distribution in the inner portion of the sample. The PTL 1 does not discuss or suggest anything about determining the average sound speed in the inner portion of the sample. That is, the invention of the PTL 1 does not aim at overcoming the problems based on the principle that is characteristic of the aforementioned PAT. In addition, the PTL 1 does not even discuss the problems. In order to determine the sound speed in the sample, it is necessary to separately obtain a photoacoustic wave signal generated by irradiating the very small absorber (disposed at the outer side of the sample) with light and a photoacoustic wave signal generated by irradiating the sample with light. As a result, it is necessary to perform at least two photoacoustic signal measurements. Therefore, a long measurement time is required until image data is formed. Further, in order to determine the sound speed distribution in the inner portion of the sample, it is necessary to obtain the aforementioned two signals from a plurality of directions. Therefore, the number of signal measurements and the measurement time are considerably increased.

The present invention is carried out on the basis of such related art and understanding of the problems. The present invention provides a photoacoustic imaging diagnosis in which the average sound speed in an inner portion of a sample can be easily calculated from a detection signal obtained when an ordinary sample measurement is performed using PAT, to obtain high-resolution image data using a measured average sound speed.

## Solution to Problem

According to the present invention, there is provided a photoacoustic imaging apparatus, **a method and a program according to the respective claims.**

## Advantageous Effects of Invention

The present invention can provide a photoacoustic imaging apparatus that can easily measure an average sound speed in an inner portion of a sample by receiving a photoacoustic wave that is generated at a surface of the sample and that propagates through the inner portion of the sample. This makes it possible to obtain high-resolution image data using an actually measured average sound speed.

## Brief Description of Drawings

   [fig. 1] Fig. 1 is a schematic view of the structure of a photoacoustic imaging apparatus according to a first embodiment of the present invention.
   [fig. 2] Fig. 2 is a flowchart illustrating an exemplary detection signal processing operation in the first embodiment of the present invention.
   [fig. 3] Fig. 3 is a schematic view of an exemplary detection signal, which is a digital signal, in the first embodiment of the present invention.
   [figs. 4A and 4B] Fig. 4A shows an image obtained in Example 1 based on the first embodiment of the present invention; and Fig. 4B shows an image obtained independently of the present invention when an average sound speed in an inner portion of a sample is assumed.
   [figs. 5A to 5C] Fig. 5A is a schematic view of the structure of a photoacoustic imaging apparatus according to a second embodiment of the present invention; Fig. 5B shows a detection signal obtained in Example 2 based on the second embodiment of the present invention; and Fig. 5C shows an image obtained in Example 2.
   [fig. 6] Fig. 6 is a schematic view of the structure of a photoacoustic imaging apparatus according to a third embodiment of the present invention.

## Description of Embodiments

The present invention will hereunder be described in detail with reference to the drawings. In general, corresponding structural components are given the same reference numerals, and the same descriptions will not be repeated.

## (1-1)th Embodiment: Photoacoustic Imaging Apparatus

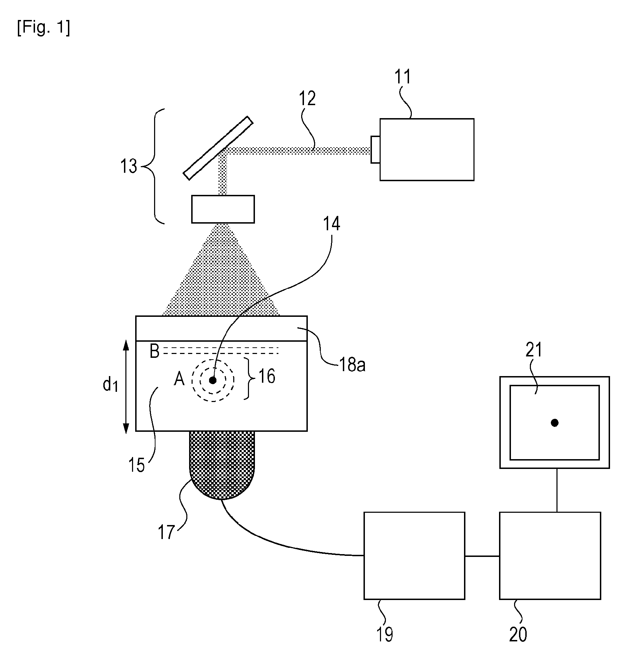

First, the structure of a photoacoustic imaging apparatus according to an embodiment will be described with reference to Fig. 1. The photoacoustic imaging apparatus according to the embodiment is an apparatus that performs imaging on optical characteristic value information of an inner portion of a sample.

A basic hardware structure of the photoacoustic imaging apparatus according to the embodiment includes a light source 11, an acoustic wave probe 17 serving as a detector, and a signal processing unit 20. Pulsed light 12 emitted from the light source 11 is guided by an optical system 13 including, for example, a lens, a mirror, and an optical fiber, and illuminates a sample 15, such as a living body. When a portion of energy of the light that has propagated through the inner portion of the sample 15 is absorbed by an optical absorber 14 (consequentially serving as a sound source), such as a blood vessel, the optical absorber 14 is thermally expanded, so that an acoustic wave 16 (typically an ultrasonic wave) is generated. The acoustic wave is also called a photoacoustic wave. The acoustic wave 16 is detected by the acoustic wave probe 17 and is converted into a digital signal by a signal acquisition unit 19, after which the digital signal is converted into image data of the sample by the signal processing unit 20.

## Light Source 11

When the sample is a living body, the light source 11 emits light having a particular wavelength that is absorbed by a particular component among components of the living body. The light source may be provided integrally with the imaging apparatus according to the embodiment, or may be provided separately from the imaging apparatus. As the light source, it is desirable to use a pulsed light source that can generate pulsed light on the order of a few nanoseconds to several hundreds of nanoseconds. More specifically, in order to efficiently generate an acoustic wave, a pulse width on the order of 10 nanoseconds is used. Although as the light source, it is desirable to user a laser because a large output is obtained, it is possible to use, for example, a light-emitting diode instead of a laser. As the laser, it is possible to use various laser types, such as a solid-state laser, a gas laser, a dye laser, and a semiconductor laser. Irradiation timing, waveforms, intensities, etc. are controlled by a controller (not shown).

In the present invention, for the wavelengths of the light source used, it is desirable to select wavelengths that are characteristically absorbed by the skin at the surface of the living body. More specifically, wavelengths in the range of from 500 nm to 1200 nm are selected. This is because, in a processing operation described below, it becomes easier to distinguish between a photoacoustic signal generated at the surface of the sample (for example, the skin) and a photoacoustic signal generated at the optical absorber (such as a blood vessel) in the inner portion of the sample.

**Optical System 13**

Although the light 12 emitted from the light source 11 is typically guided to the sample by optical components, such as a lens and a mirror, it is possible to propagate the light using, for example, a light guide such as an optical fiber. The optical system 13 includes, for example, a mirror that reflects the light and a lens that converges and enlarges the light and changes the form of the light. Any optical component may be used as long as it causes the light 12 emitted from the light source to illuminate the sample 15 in a predetermined form. In general, it is better to increase the area of the light to a certain extent rather than converging the light with a lens from the viewpoints of increased safety and an increased diagnosis area of the living body. In addition, it is desirable that the area of the sample irradiated with the light be movable. In other words, it is desirable that the imaging apparatus according to the present invention be formed so that the light generated from the light source is movable along the sample. When the light is movable along the sample, it is possible to irradiate a wider range with the light. Further, it is more desirable that the area of illumination of the sample with the light (that is, the light that illuminates the sample) be movable in synchronism with the acoustic wave probe 17. Examples of a method of moving the area of irradiation of the sample with the light are a method using, for example, a movable mirror and a method that mechanically moves the light source itself.

**Sample and Optical Absorber**

The sample and the optical absorber do not constitute a part of the imaging apparatus according to the present invention, but will be described below. The photoacoustic imaging apparatus according to the present invention is primarily provided for, for example, diagnosing blood vessel diseases, malignant tumors of human beings and animals, etc., and observing a chemical treatment process. As the sample, the living body, more specifically, the breast, the fingers, the hands, the legs, etc. of human beings and animals may be diagnosed. The optical absorber 14 in the inner portion of the sample has a relatively high absorption coefficient in the inner portion of the sample. For example, if the object to be measured is a human body, oxygenated, deoxygenated hemoglobin, blood vessels including large amounts of these substances, and malignant tumors including a large number of new blood vessels correspond to the optical absorber. Although not shown, melanin, which exists near the surface of the skin, exists as an optical absorber at the surface of the sample. In the present invention, "living body information" refers to a generating source distribution of acoustic waves generated by the light irradiation, and indicates initial sound pressure distribution in the living body, optical energy absorption density derived therefrom, and a concentration distribution of substances making up tissues obtained from these items of information. For example, the concentration distribution of substances include oxygen saturation. These items of information that have been subjected to imaging are called image data.

**Acoustic Wave Probe 17**

The acoustic wave probe 17, which is a detector that detects acoustic waves, generated at the surface and the inner portion of the sample using the pulsed light, detects the acoustic waves and converts the acoustic waves into electric signals which are analog signals. The acoustic wave probe 17 may hereunder be simply referred to as a "probe" or a "transducer". Any photoacoustic wave detector can be used as long as it can detect acoustic signals, such as a transducer making use of piezoelectric phenomena, a transducer making use of resonance of light, and a transducer making use of changes in capacity. The probe 17 in the embodiment typically includes a plurality of receiving elements that are one-dimensionally or two-dimensionally disposed. By using multidimensionally disposed elements in this way, it is possible to detect the acoustic waves simultaneously at a plurality of locations, to reduce detection time, and to reduce influences of, for example, vibration of the sample.

**Flat Plate 18**

In the embodiment, the sample 15 is compressed and secured by a flat plate 18a. The light irradiation is performed through the flat plate 18a. The flat plate 18a holds the sample, and is formed of an optically transparent material for transmitting the light therethrough. Typically, acryl is used. When it is also necessary to transmit acoustic waves, in order to suppress reflection, it is desirable to use a material whose acoustic impedance does not differ much from that of the sample. When the sample is a living body, for example, polymethylpentene is typically used. Although the flat plate 18a may be formed to any thickness as long as the flat plate 18a is strong enough to suppress deformation of the flat plate 18a when it holds the sample, the thickness is typically on the order of 10 mm. Although the flat plate 18a may be of any size as long as it can hold the sample, the size of the flat plate 18a is basically the same as the size of the sample.

Although, in Fig. 1, the flat plate 18a is only provided at a light irradiation side, and the probe 17 directly contacts the sample 15, a flat plate may be provided along the entire surface of the probe 17. That is, the sample may be compressed and secured from both sides by a first flat plate and a second flat plate that are disposed substantially parallel to each other. The light source is disposed at the side of the first plate 18a, and the probe 17 is disposed at the side of the second plate (not shown in Fig. 1).

**Signal Acquisition Unit 19**

It is desirable that the imaging apparatus according to the embodiment include the signal acquisition unit 19 that amplifies the electric signals obtained from the probe 17 and converts the electric signals from analog signals to digital signals. The signal acquisition unit 19 is typically formed by, for example, an amplifier, an A/D converter, and a field programmable gate array (FPGA) chip. When there are a plurality of detection signals obtained from the probe, it is desirable that the plurality of signals be processed at the same time. This makes it possible to reduce the time until images are formed.

In the specification, the term "detection signal" is a concept referring to the analog signal obtained from the probe 17 and the digital signal obtained thereafter after the analog-to-digital conversion. In addition, the detection signal is also called a "photoacoustic signal".

**Signal Processing Unit 20**

The signal processing unit 20 calculates the average sound speed in the inner portion of the sample. This calculation is a characteristic feature of the present invention. Using the detection signal, obtained from the acoustic wave generated at the inner portion of the sample, and the above calculated average sound speed, image data of the inner portion of the sample is generated (that is, images are reconstructed). Although described in more detail later, that the average sound speed is calculated on the basis of the detection signal obtained from the acoustic wave (first acoustic wave) generated at the surface of the sample and propagated through the inner portion of the sample is a characteristic feature of the present invention. In actually calculating the average sound speed on the basis of the acoustic wave propagating through the inner portion of the sample, a calculated value is an actual measurement value of the average sound speed in the inner portion of the sample. Since the acoustic wave is generated at both the surface and the inner portion of the sample by irradiating the sample with the light, when the signal processing is performed with some thought, it is possible to calculate the average sound speed and generate the image data of the inner portion of the sample by one light irradiation operation.

In the signal processing unit 20, for example, a workstation is typically used. The calculation of the average sound speed, the image reconstruction processing, etc. are performed on the basis of a previously programmed software. For example, the software used in the workstation includes two modules, that is, a signal processing module for determining the average sound speed from the detection signals and for reducing noise and an image reconstruction module for the image reconstruction. In the photoacoustic tomography, ordinarily, as a preprocessing operation performed prior to the image reconstruction, for example, the noise reduction is performed on a signal received at each location. It is desirable that such a preprocessing operation be performed with the signal processing module. In the image reconstruction module, image data is formed by the image reconstruction. As an image reconstruction algorithm, for example, a back projection method in a Fourier domain or a time domain ordinarily used in tomographic technology is applied. Exemplary image reconstruction methods using PAT typically include a Fourier transformation method, a universal back projection method, and a filtered back projection method. Since these methods also use the average sound speed as a parameter, it is desirable to actually measure the average sound speed precisely in the present invention.

Depending on the circumstances, the signal acquisition unit 19 and the signal processing unit 20 may be integrated to each other. In this case, it is possible to generate the image data of the sample not only by a software processing operation performed at the workstation, but also by a hardware processing operation.

**Display Apparatus 21**

A display apparatus 21 displays the image data output by the signal processing unit 20. For example, a liquid crystal display apparatus is typically used as the display apparatus 21. The display apparatus 21 may be provided separately from a diagnostic imaging apparatus according to the present invention.

**Processing of Detection Signal**

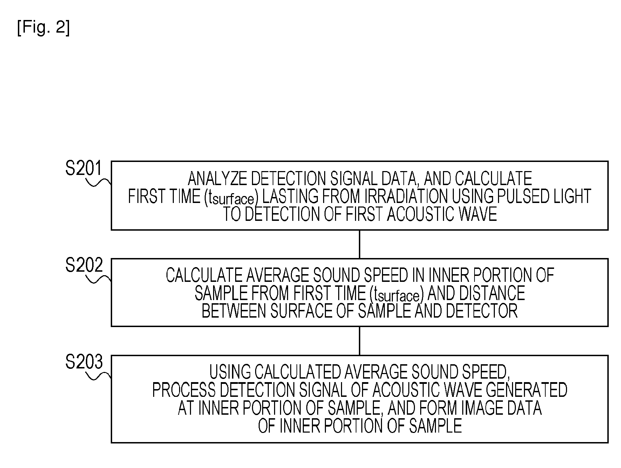

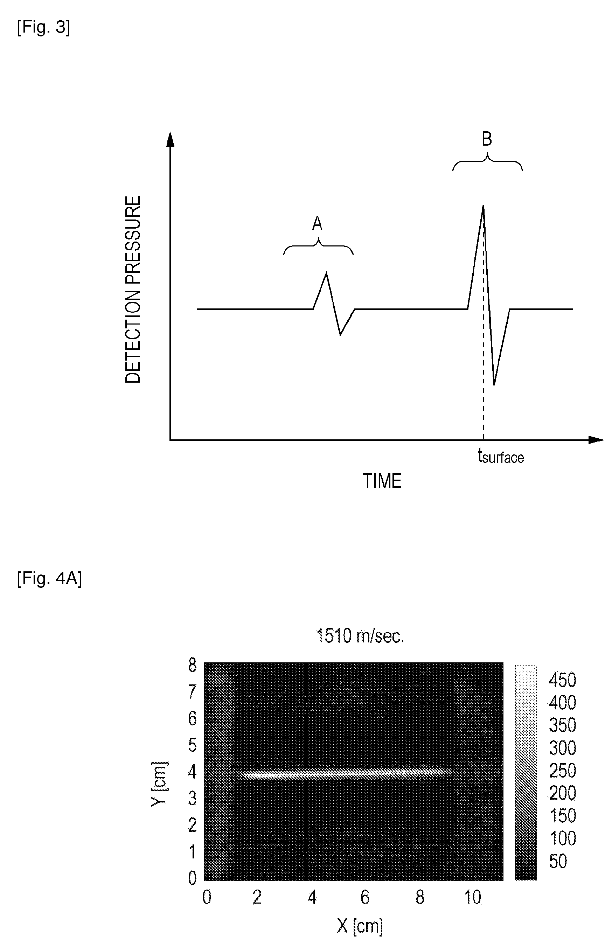

Next, the calculation of the average sound speed in the inner portion of the sample performed by the signal processing unit 20 will be described with reference to Figs. 2 and 3. The numbers below correspond to the numbers indicating the processing steps in Fig. 2.

Processing Step (1) (S201) is a step in which detection signal data is analyzed to calculate a first time ($t_{surface}$) lasting from the irradiation with the pulsed light to the detection of the first acoustic wave.

The digital signal (see Fig. 3) obtained from the signal acquisition unit 19 shown in Fig. 1 is analyzed to specify the first time ($t_{surface}$). Ordinarily, when the sample 15 is irradiated with pulsed light, as shown in Fig. 3, a plurality of signals having N-type forms are observed. These signals are primarily detection signals obtained from photoacoustic waves generated at the optical absorber 14 existing in the inner portion of the sample (such as blood in the case of a living body) and at the surface of the sample (such as pigments on the surface of the skin in the case of a living body). The reason a relatively large photoacoustic wave is generated at the surface of the sample irradiated with light is that, even if the optical absorption coefficient of the surface of the sample is small, the intensity of the light used for irradiating the surface of the sample is larger than that at the inner portion of the sample. In the example shown in Fig. 3, reference character A denotes a detection signal obtained from the photoacoustic wave generated from the optical absorber 14 existing in the inner portion of the sample, and reference character B denotes a detection signal obtained from the photoacoustic wave generated at the surface of the sample. In Fig. 3, a time of pulsed light irradiation t is 0. If the light speed and the size of the sample are considered, it can be said that at the same time that the irradiation with the pulsed light is performed, the photoacoustic waves are simultaneously generated from their respective locations. That is, the time of propagation of the pulsed light through the inner portion of the sample is so small as to be negligible compared to the propagation time of the acoustic waves (that is, the measurement time of the acoustic waves).

A method of distinguishing between the photoacoustic signal A generated in the inner portion of the sample and the photoacoustic signal B generated at the surface of the sample will hereunder be described. In the embodiment, the first acoustic wave is generated from the surface of the sample secured to the compression plate 18a. When, as shown in Fig. 1, the probe 17 is disposed at a surface of the sample at a side opposite to an optical irradiation area, the photoacoustic wave generated at the surface of the sample reaches the probe 17 later than the photoacoustic wave generated from the optical absorber 14 in the inner portion of the sample. By making use of this characteristic, it is possible to easily distinguish it from other photoacoustic signals (for example, A in Fig. 3). That is, it can be determined that the large photoacoustic wave that is detected last is the first acoustic wave.

If the surface of the sample that is irradiated with light is formed into a flat surface as shown in Fig. 1 by, for example, the flat plate as in the embodiment, the photoacoustic wave generated from the surface of the sample propagates like a plane wave. In contrast, since the optical absorber in the inner portion of the sample is sufficiently smaller than the optical irradiation area, the photoacoustic wave 16 often propagates like a spherical wave. Broken lines A and B in Fig. 1 represent wave surfaces of the photoacoustic waves. Considering the differences in such propagation characteristics, it is desirable to perform signal processing for intensifying the detection signal obtained from the acoustic wave generated at the surface of the sample. This makes it possible to precisely detect the first acoustic wave, so that the precision with which the average sound speed is calculated is increased.

A specific example of the processing is described below. For example, detection signals detected by the plurality of receiving elements can be compared with each other. When the plurality of receiving elements contact the surface of the sample, a plane wave reaches the plurality of receiving elements at substantially the same time. However, a spherical wave reaches the plurality of receiving elements at different times. Therefore, such a comparison makes it possible distinguish the acoustic wave generated from the surface of the sample from the acoustic wave generated from the inner portion of the sample.

All of the detection signals detected at the respective receiving elements may be averaged at all of the receiving elements. The term "all of the detection signals" means all of the detection signals obtained by receiving both the photoacoustic waves at the surface and the inner portion of the sample. In this processing, at the plurality of receiving elements, the detection signals originating from the acoustic waves from the surface of the sample and detected at the same time are strengthened, and the detection signals originating from the acoustic waves from the inner portion of the sample and detected at different times are weakened. Even for signals including, for example, noise, only the photoacoustic signals generated at the surface of the sample can be specified.

As the method of specifying the detection signal obtained from the acoustic wave generated at the surface of the sample, a method that makes use of pattern matching may be used. For example, the pattern matching is performed to specify an N-type detection signal that is characteristic of the acoustic wave generated from the surface of the sample, and a time position of the specified N-type detection signal is defined as a first time $t_{surface}$. More specifically, a time position of the N-type signal at a minimum peak or a maximum peak is defined as $t_{surface}$. After a detection signal other than that obtained from the acoustic wave generated from the surface of the sample is reduced using the above-described method, for example, a method of detecting the peak of the N-type detection signal obtained from the acoustic wave generated at the surface of the sample by searching for a maximum and a minimum value may be used. Even in this method, the time position of the N-type signal at the minimum peak or the maximum peak is defined as $t_{surface}$. By, for example, the aforementioned methods, the first time t surface can be calculated. Of the time positions at the maximum peak and the minimum peak of the N-type detection signal obtained from the acoustic wave generated from the surface of the sample, which of these is to be the first time depends upon the characteristics of the probe.

Processing Step (2) (S202) is a step in which the average sound speed in the inner portion of the sample is calculated from the first time ($t_{surface}$) and the distance between the surface of the sample and the detector.

An average velocity $c_{average}$ of the sample is calculated from the first time ($t_{surface}$) obtained by the aforementioned processing, and a distance $d_1$ between the surface of the sample at a light irradiation position and the probe. Here, the average velocity c $_{average}$ can be obtained by a simple Expression (1) given below:

$$c_{average} = a_1/t_{surface} \quad (1)$$

In the embodiment in which the probe 17 is provided directly on the sample 15, the first acoustic wave propagates only through the inner portion of the sample over the first time ($t_{suface}$), so that the average sound speed can be calculated using the aforementioned expression. This means that the average sound speed of the sample can be actually measured by using the acoustic wave generated at the surface of the sample and propagated through the inner portion of the sample.

In the embodiment shown in Fig. 1, the distance $d_1$ is the distance from the surface of the sample, secured to the first plate 18a, to the probe. The distance $d_1$ may be included as a known value in the signal processing module in the embodiment, or may be measurable by position control of the movable plate 18a. The distance $d_1$ may be measurable with any distance sensor, or may be obtained from a result of measurement of the shape of the sample performed with, for example, a camera that can perform imaging on the entire sample.

Processing Step (3) (S203) is a step in which, using the calculated average sound speed, the detection signal of the acoustic wave generated at the inner portion of the sample is processed to form image data of the inner portion of the sample.

Using the average sound speed $c_{average}$ obtained by the processing step (2), and a plurality of digital detection signals output from the signal acquisition unit 19, the image reconstruction processing is performed, so that data related to the optical characteristics of the sample is formed. For example, back projection in a Fourier domain or a time domain used in a general photoacoustic tomography is suitable.

By performing the above-described steps, it is possible to easily calculate the average sound speed using only the signals obtained by irradiating the sample with light, and to obtain an image whose resolution is not reduced due to a difference in sound speed by using the average sound speed in the image reconstruction.

**Example 1**

An exemplary imaging apparatus using photoacoustic tomography to which the embodiment is applied will be described using the schematic view of the apparatus shown in Fig. 1. In the example, as a light source 11, a Q switch YAG laser generating pulsed light of approximately 10 nanoseconds at a wavelength of 1064 nm was used. Energy of a light pulse emitted from pulsed laser light 12 was 0.6 J. Using an optical system 13, such as a mirror and a beam expander, the pulsed light was expanded to a radius of approximately 2 cm. A phantom or a simulation of a living body was used as a sample 15. For the phantom, 1% Intralipid with gelatin was used. The average sound speed in the phantom was a known value of 1512 m/sec. The size of the phantom was such that its width was 12 cm, its height was 8 cm, and its depth was 4 cm. As an optical absorber 14, a black rubber wire having a diameter of 0.03 cm was buried near the center in the phantom. As a result of interposing the phantom between a probe 17 and an acrylic plate 18a having a thickness of 1 cm, a thickness ($d_1$) in a depth direction of the phantom obtained using a distance sensor was 4 cm. The phantom having such a prescribed thickness in the depth direction was irradiated with the pulsed light 12. As the acoustic wave probe 17, an ultrasonic transducer formed of lead zirconate titanate (PZT) was used. The transducer was a two-dimensional array type and square-shaped, with the number of elements being 18 * 18, and the element pitch being 2 mm. The width of each element was approximately 2 mm. In synchronism with a light irradiation area, the photoacoustic probe was movable in a direction of a plane of the phantom, and was capable of performing imaging on a large area.

As shown in Fig. 1, when a plane at one side of the phantom (that is, a side of the phantom opposite to the probe) was irradiated with the pulsed light, a photoacoustic wave generated by optical absorption at a surface of the phantom at a light illumination side and a photoacoustic wave generated by absorbing by a rubber wire light scattered in the phantom were generated. Using the ultrasonic transducer, the photoacoustic waves were received at the same time by 324 channels. Using a signal acquisition unit 19 including an amplifier, an AD converter, and a FPGA, digital data of a photoacoustic signal at each channel was obtained. For improving a S/N ratio of each signal, irradiation with laser was performed 36 times, to average all of the obtained detection signals in terms of time. Thereafter, the obtained pieces of digital data were transferred to a workstation (WS) serving as a signal processing unit 20, and were stored in the WS. Next, with respect to the stored received data, the received pieces of data for all of the elements were averaged. The results were as follows. Since, for the photoacoustic signals generated from the optical absorber in the phantom, detection times for the respective received pieces of data for the respective elements differed from each other, these photoacoustic signals were considerably reduced due to the averaging. In contrast, since, for the photoacoustic signals generated at the surface of the sample, detection times for the respective received pieces of data for the respective elements were substantially the same, these photoacoustic signals were intensified with respect to other signals by the averaging. Next, for the average signal of all of the detection signals, a minimum signal value was detected, so that the time corresponding to the minimum value was defined as a detection time of the photoacoustic signals generated at the surface of the sample. As a result, the obtained detection time was approximately 26.5 microseconds. The average sound speed in the phantom obtained from the detection time and the thickness of the phantom in the depth direction was 1510 m/sec. This substantially matched the actual sound speed in the phantom.

After performing a noise reduction operation on the detection signals by discrete wavelet transformation, the image reconstruction was performed using the calculated average sound speed in the phantom. Here, using the universal back projection method, which is a time domain method, volume data was formed. A voxel interval used here was 0.05 cm. An imaging range was 11.8 cm * 11.8 cm * 4.0 cm. An exemplary image obtained at this time is shown in Fig. 4A.

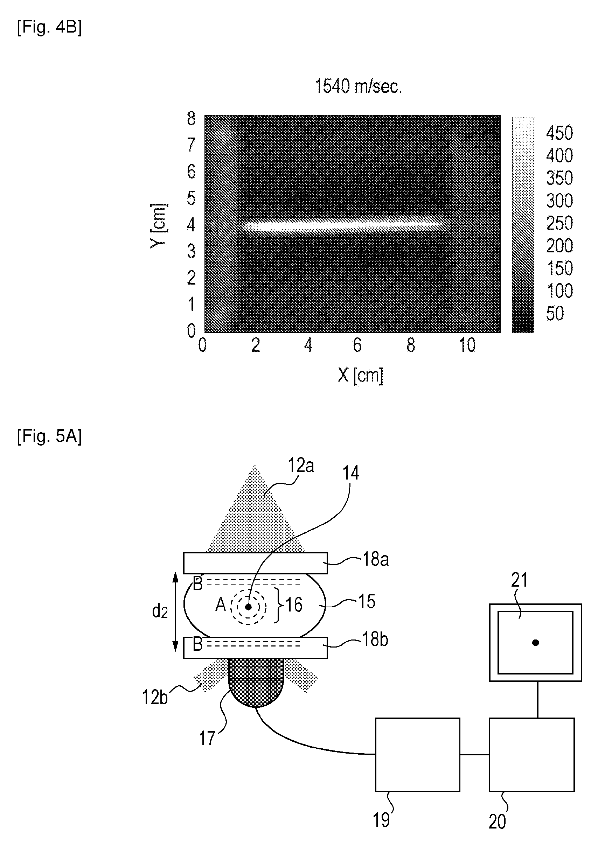

Next, without measuring the average sound speed in the phantom, it was assumed that the average sound speed in the phantom was equal to 1540 m/sec, corresponding to the average sound speed in a living body, and the image reconstruction was performed again using the pieces of detection signal data stored in the WS. An exemplary image obtained at this time is shown in Fig. 4B.

Comparing Figs. 4A and 4B, it is obvious that a width of an initial sound pressure, generated from the rubber wire, for the image subjected to the image reconstruction at an average sound speed of 1510 m/sec is smaller than that for the image subjected to the image reconstruction at an average sound speed in 1540 m/sec. In addition, the image subjected to the image reconstruction at an average sound speed in 1510 m/sec has less blur. In other words, its resolution is improved. Accordingly, when the average sound speed in the sample cannot be estimated, in the present invention, a reduction in the resolution can be suppressed by actually measuring the average sound speed in the sample.

**(1-2)th Embodiment**

In the (1-1)th embodiment, the probe 17 is directly set at the sample 15. However, in this embodiment, it is assumed that a sample is compressed and secured at respective sides thereof by a first flat plate and a second flat plate disposed substantially parallel to each other. The probe 17 is set at a surface of the second flat plate. In this case, an acoustic wave generated at a surface of the sample secured by the first plate is defined as a first acoustic wave. Since the first acoustic wave is received by the probe 17 not only after it propagates through an inner portion of the sample but also after it propagates through the second plate, the first time ($t_{surface}$) explained in the (1-1)th embodiment is no longer the time taken for the first acoustic wave to pass through the inner portion of the sample. Accordingly, when an area other than the inner portion of the sample is included in a path that the first acoustic wave, indispensable to the calculation of the average sound speed, passes until it reaches the probe 17, it is necessary to determine the average sound speed taking this into consideration.

More specifically, from the first time ($t_{surface}$), the time required for the first acoustic wave to pass through the second plate is subtracted, so that a second time required for the first acoustic wave to pass through the inner portion of the sample is calculated. "The time required for the first acoustic wave to pass through the second plate" may be included in the signal processing module as a known value from the thickness of and the sound speed (a characteristic value obtained from a material) in the second plate.

A propagation distance of the first acoustic wave in the inner portion of the sample can be equated with a distance $d_2$ between the first plate and the second plate. If, in the Expression (1), the second time is substituted for the first time ($t_{surface}$) and $d_2$ is substituted for the distance $d_1$, the average sound speed in the sample can be actually measured.

**Second Embodiment**

In the first embodiment, the average sound speed is calculated by using only the acoustic wave (first acoustic wave) generated from one location. In a second embodiment, the average sound speed is calculated by using acoustic waves generated at a plurality of surfaces of a sample. That is, the average sound speed is calculated from detection signals obtained from a first acoustic wave and a second acoustic wave generated at a surface of the sample that differs from the surface of the sample where the first acoustic wave is generated. This will hereunder be described on the basis of Example 2.

**Example 2**

Example 2 in which, in an imaging apparatus using photoacoustic tomography, laser was used for irradiation from two directions will be described with reference to Fig. 5A. The basic structure of the imaging apparatus according to Example 2 was the same as that of the imaging apparatus according to Example 1 except that a sample 15 was interposed between two plates 18a and 18b, to regulate the size of the sample. That is, the size of the sample was regulated by controlling the interval between the plates. The thickness of each plate was 1 cm. The sample could be irradiated through the plate 18b from a side of a probe 17 and in a direction that is the same as that in Example 1. A phantom used was one having titanium oxide and ink mixed with urethane rubber. The size of the phantom was such that its width was 8 cm, its height was 8 cm, and its depth was 5 cm. An optical absorber having a columnar shape that was 0.5 cm in diameter and having a high absorption coefficient with respect to that of a base material as a result of mixing a large amount of ink was buried in the phantom. As a result of interposing the phantom between the two plates, the thickness of the phantom in the depth direction obtained by a distance sensor was 4.9 cm. The phantom whose thickness in the depth direction was regulated in this way was irradiated with pulsed light 12 from both sides thereof. The pulsed light was emitted in synchronism from two light sources. As the probe 17, an ultrasonic transducer formed of lead zirconate titanate (PZT) was used. The transducer was a two-dimensional array type and square-shaped, with the number of elements being 15 * 23 and the element pitch being 2 mm. The width of each element was approximately 2 mm.

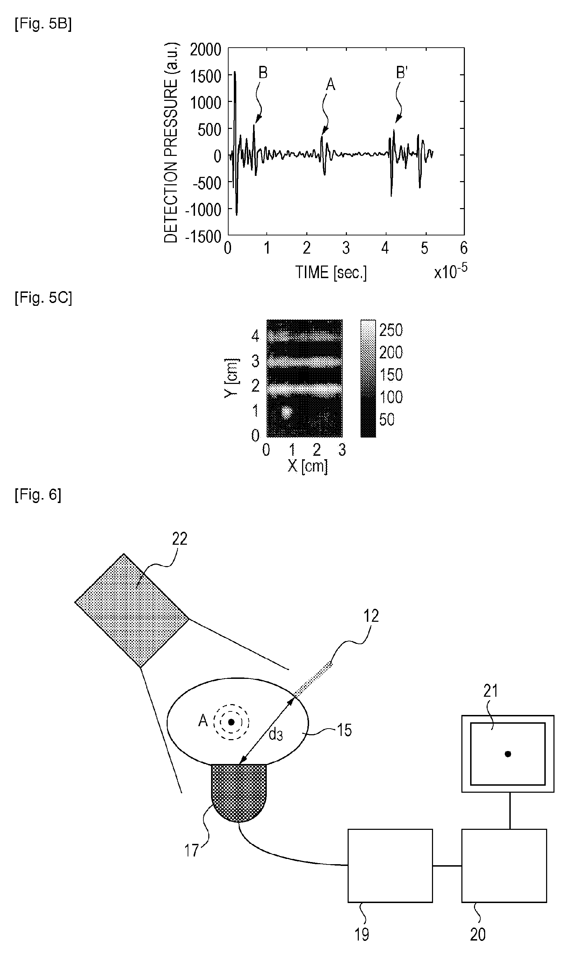

As a result of irradiating such a phantom with the light, the first acoustic wave and the second acoustic wave were generated from surfaces of the phantom secured by the first plate 18a and the second plate 18b, and an acoustic wave was also generated from the optical absorber in the phantom. The ultrasonic transducer received these acoustic waves simultaneously at 345 channels. Then, using a signal acquisition unit 19 including an amplifier, an AD converter, and an FPGA, items of digital data of the photoacoustic signals at all of the channels were obtained. An exemplary received signal is shown in Fig. 5B. Reference character B in Fig. 5B represents a detection signal of the acoustic wave (second acoustic wave) generated at a probe-side surface of the phantom. Reference character A represents a detection signal of the photoacoustic wave generated at the optical absorber in the phantom. Reference character B' represents a detection signal of the photoacoustic wave (first acoustic wave) generated at the surface of the phantom at a side opposite to the probe as a result of the irradiation with light.

The first acoustic wave passed through an inner portion of the phantom and the second plate 18b, and reached the probe 17. In contrast, the second acoustic wave did not pass through the inner portion of the phantom. The second acoustic wave passed only through the second plate 18b, and reached the probe 17. The time it took the first acoustic wave to pass through the second plate and the time it took the second acoustic wave to pass through the second plate were the same. Accordingly, if the difference between times of detections of the first and second acoustic waves, that is, a time difference of 35.8 microsec between B and B' was calculated, the time taken for the first acoustic wave to pass through only the inner portion of the phantom was obtained. When the average sound speed in the phantom was calculated by dividing 4.9 cm, that is, the distance between the surfaces of the phantom, by this time, it was 1370 m/sec.

Next, using the average sound speed calculated from the received pieces of digital data of the photoacoustic signals at all of the channels, image reconstruction was performed. Here, using the universal back projection method, which is a time domain method, volume data was formed. A voxel interval used at this time was 0.025 cm. An imaging range was 3.0 cm * 4.6 cm * 4.9 cm. An exemplary image obtained at this time is shown in Fig. 5C. In the image, the optical absorber disposed in the phantom was subjected to imaging. Positions thereof matched the actual position of the optical absorber in the phantom. When the image of the optical absorber was analyzed, the resolution was approximately 2 mm, which substantially matched a theoretical resolution limit of 2 mm. Accordingly, when the average sound speed in the sample was not known, an image whose resolution was not reduced could be obtained by using the present invention.

In such an embodiment, the average sound speed can be calculated by dividing the distance between the surface of the sample where the first acoustic wave is generated and the surface of the sample where the second acoustic wave is generated by the difference between the time of detection of the first acoustic wave and the time of detection of the second acoustic wave. This calculation method is effective when a path taken by the first acoustic wave and the second acoustic wave until they reach the probe 17 is common, and the difference between the lengths of the path taken by the first and second acoustic waves correspond to the length of the inner portion of the sample.

In the embodiment, since the average sound speed can be calculated by using the difference between the time of detection of the first acoustic wave and the time of detection of the second acoustic wave, it is not necessary to accurately know the timing of irradiation using pulsed light as in the first embodiment. Therefore, this embodiment is advantageous from the viewpoint that it is not influenced by measurement errors caused by external factors such as instability of a light source system. Although, in this embodiment, the second plate 18b is not required, even if the second plate 18b exists along the entire surface of the probe 17, the time taken for the acoustic wave to pass through the second plate 18b is canceled by an operation for eliminating the time difference. Therefore, correction as in the (1-2)th embodiment is not required.

In the embodiment, it is not necessary to irradiate both sides of the sample as in Example 2. Only one side of the sample may be irradiated as in the first embodiment. When one side is irradiated, light illuminating the surface of the sample secured to the first plate may propagate through the inner portion of the sample while being attenuated, and reach the surface of the sample at the opposite side. In this case, a very weak second acoustic wave may be generated from the surface of the sample at the opposite side. However, if the thickness of the sample is on the order of 4 cm, even from the viewpoint of reliably eliminating the time difference, it is necessary for the second acoustic wave to have a certain intensity. Therefore, it is desirable to irradiate the sample from both sides.

In the embodiment, the distance sensor in Example 2 is not required. The distance between the surface of the sample where the first acoustic wave is generated and the surface of the sample where the second acoustic wave is generated may be a known distance.

## Third Embodiment

Although, in the first and second embodiments, at least one flat plate 18a is used to secure the sample, the present invention is not limited thereto. Example 3 in which measurements are carried out by setting the probe 17 at the sample whose shape is not regulated by a plate will be described below.

### Example 3

Example 3 will be described with reference to a schematic view of an apparatus shown in Fig. 6. The basic structure of the apparatus according to this example was similar to those of the apparatuses of Examples 1 and 2. However, the apparatus according to Example 3 included a camera 22 measuring the shape of a sample. Using the camera 22, the sample was captured. From an analysis of an image thereof, a distance $d_3$ between the probe 17 and a light irradiation area was calculated. Since an acoustic wave generated at a surface of the sample passed only through an inner portion of the sample until it reached the probe 17, it was possible to use Expression (1) indicated in the (1-1)th embodiment. In place of the distance $d_1$, the distance $d_3$ was input, to calculate the average sound speed. Then, using the calculated average sound speed, information regarding the living body of the sample was reconstructed. Accordingly, even if the shape of the sample was complicated, as long as the shape of the sample could be confirmed using the camera, it was possible to calculate the average sound speed in the sample and obtain an image whose resolution was not reduced.

### Fourth Embodiment

The present invention is carried out by executing the following operations. That is, a software (program) for realizing the functions in the above-described embodiments is supplied to a system or an apparatus through a network or various storage media, and the system or a computer (or a CPU, MPU, etc.) of the apparatus reads out and executes the program.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

### Reference Signs List

11    Light source
17    Acoustic wave probe
18    Flat plate
20    Signal processing unit

### Claims

1.    A photoacoustic imaging apparatus comprising:

a detector (17) configured to output detection signals by detecting acoustic waves generated at surfaces and an inner portion of a sample by irradiating the sample with light; and
a signal processing unit (20) configured to generate image data using the detection signals,
wherein the detector is configured to output a first detection signal by detecting a first acoustic wave which is generated at a first surface of the sample and propagated through the inner portion of the sample,
wherein the signal processing unit is configured to calculate an average sound speed in the inner portion of the sample by using the first detection signal and to generate the image data using the average sound speed and the detection signals.

2.    The photoacoustic imaging apparatus according to Claim 1, wherein the signal processing unit is configured to calculate the average sound speed in the inner portion of the sample from a distance between the first surface of the sample and the detector and from a time from when the sample is irradiated with the light to when the first acoustic wave.

3.    The photoacoustic imaging apparatus according to Claim 1, wherein the detector is configured to output a second detection signal by detecting a second acoustic wave generated at a second surface of the sample different from the first surface of the sample from among the surfaces of sample, wherein the signal processing unit is configured to calculate the average sound speed in the inner portion of the sample from the first detection signal and the second detection signal.

4.    The photoacoustic imaging apparatus according to Claim 3, wherein the signal processing unit is configured to calculate the average sound speed in the inner portion of the sample by dividing a distance between the first surface of the sample and the second surface of the sample by a difference between a detection time of the first acoustic wave and a detection time of the second acoustic wave.

5.    The photoacoustic imaging apparatus according to any one of Claims 1 to 4, wherein the signal processing unit is configured, based on differences in propagation characteristics between the acoustic waves generated at the surfaces of the sample and the acoustic wave generated at the inner portion of the sample, to perform processing for intensifying the detection signals obtained from the acoustic waves generated at the surfaces of the sample, to specify the detection signals obtained from the acoustic waves generated at the surfaces of the sample, and to calculate the average sound speed in the inner portion of the sample from the detection signals generated at the surfaces of the sample.

**6.** The photoacoustic imaging apparatus according to Claim 5, wherein the detector includes a plurality of receiving elements, and the processing corresponds to averaging items of data of all of the detection signals detected at the plurality of receiving elements of the detector.

**7.** The photoacoustic imaging apparatus according to Claim 1, further comprising: a first flat plate and a second flat plate disposed substantially parallel to each other and configured to hold the sample from both sides of the sample, and a first light source configured to irradiate the first surface with light through the first flat plate, wherein the detector is disposed so as to acoustically contact the second flat plate and output the first detection signal by detecting the first acoustic wave generated at the first surface of the sample held by the first flat plate.

**8.** The photoacoustic imaging apparatus according to Claim 7, wherein the signal processing unit is configured to calculate the average sound speed in the inner portion of the sample from a distance between the detector and the first surface of the sample held to the first flat plate and from a first time from when the sample is irradiated with the light to when the first acoustic wave is detected.

**9.** The photoacoustic imaging apparatus according to Claim 8, wherein the signal processing unit is configured to calculate a second time required for the first acoustic wave to propagate through the inner portion of the sample by subtracting a time that it takes the first acoustic wave to propagate through the second flat plate from the first time, and to calculate the average sound speed in the inner portion of the sample by dividing a distance between the first flat plate and the second flat plate by the second time.

**10.** The photoacoustic imaging apparatus according to Claim 7, wherein the signal processing unit is configured to calculate the average sound speed in the inner portion of the sample based on a difference between a detection time of the first acoustic wave and a detection time of a second acoustic wave and a distance between the surface of the sample where the first acoustic wave is generated and the surface of the sample where the second acoustic wave is generated, the first and second acoustic waves being generated from the surfaces of the sample held by the first and second flat plate, the detection times being detected by the detector.

**11.** The photoacoustic imaging apparatus according to any one of Claims 7 to 10, further comprising:

a second light source different from the first light source,
wherein the second light source is configured to irradiate the second surface with light from the second light source through the second flat plate in synchronism with the first light source.

**12.** A photoacoustic imaging method in which detection signals are output by detecting acoustic waves generated at surfaces and an inner portion of a sample by irradiating the sample with light, and image data is generated using the detection signal, the method comprising the steps of:

calculating an average sound speed in the inner portion of the sample by using a first detection signal of a first acoustic wave generated at a first surface of the sample and propagated through the inner portion of the sample, and generating the image data using the average sound speed and the detection signals.

**13.** A program that when executed on a computer, causes the computer to perform the "calculating" and "generating" steps of the photoacoustic imaging method according to Claim 12.

**Patentansprüche**

**1.** Fotoakustische Abbildungsvorrichtung mit:

einem Detektor (17), der konfiguriert ist, Erfassungssingale durch Erfassen von akustischen Wellen, die an Oberflächen und an einem inneren Abschnitt einer Probe durch Bestrahlen der Probe mit Licht erzeugt werden, auszugeben; und
einer Signalverarbeitungseinheit (20), die konfiguriert ist, Bilddaten unter Verwendung der Erfassungssingale zu erzeugen, wobei
der Detektor konfiguriert ist, ein erstes Erfassungssignal durch Erfassen einer ersten akustischen Welle, die an einer ersten Oberfläche der Probe erzeugt wird und durch den inneren Abschnitt der Probe propagiert ist, auszugeben, wobei
die Signalverarbeitungseinheit konfiguriert ist, eine durchschnittliche Schallgeschwindigkeit in dem inneren Abschnitt der Probe unter Verwendung des ersten Erfassungssignals zu berechnen und die Bilddaten unter Verwendung der durchschnittlichen Schallgeschwindigkeit und der Erfassungssignale zu erzeugen.

**2.** Fotoakustische Abbildungsvorrichtung nach Anspruch 1, wobei die Signalverarbeitungseinheit konfiguriert ist, die durchschnittliche Schallgeschwindigkeit in dem inneren Abschnitt der Probe aus einem Abstand zwischen der ersten Oberfläche der Probe und dem Detektor und aus einer Zeit von dann, wenn die Probe mit dem Licht bestrahlt wird, bis dann, wenn die erste akustische Welle erfasst wird, zu berechnen.

3. Fotoakustische Abbildungsvorrichtung nach Anspruch 1, wobei der Detektor konfiguriert ist, ein zweites Erfassungssignal durch Erfassen einer zweiten akustischen Welle, die an einer zweiten Oberfläche der Probe, die unter den Oberflächen der Probe verschieden von der ersten Oberfläche der Probe ist, erzeugt wird, auszugeben, wobei die Signalverarbeitungseinheit konfiguriert ist, die durchschnittliche Schallgeschwindigkeit in dem inneren Abschnitt der Probe aus dem ersten Erfassungssignal und dem zweiten Erfassungssignal zu berechnen.

4. Fotoakustische Abbildungsvorrichtung nach Anspruch 3, wobei die Signalverarbeitungseinheit konfiguriert ist, die durchschnittliche Schallgeschwindigkeit in dem inneren Abschnitt der Probe durch Teilen eines Abstands zwischen der ersten Oberfläche der Probe und der zweiten Oberfläche der Probe durch eine Differenz zwischen einer Erfassungszeit der ersten akustischen Welle und einer Erfassungszeit der zweiten akustischen Welle zu berechnen.

5. Fotoakustische Abbildungsvorrichtung nach einem der Ansprüche 1 bis 4, wobei die Signalverarbeitungseinheit konfiguriert ist, basierend auf Differenzen in Propagationscharakteristiken zwischen den akustischen Wellen, die an den Oberflächen der Probe erzeugt werden, und der akustischen Welle, die an dem inneren Abschnitt der Probe erzeugt wird, ein Verarbeiten zum Intensivieren der Erfassungssignale, die von den akustischen Wellen, die an den Oberflächen der Probe erzeugt werden, erhalten werden, durchzuführen, um die Erfassungssignale, die von den akustischen Wellen, die an den Oberflächen der Probe erzeugt werden, zu spezifizieren, und die durchschnittliche Schallgeschwindigkeit in dem inneren Abschnitt der Probe aus den Erfassungssignalen zu berechnen, die an den Oberflächen der Probe erzeugt werden.

6. Fotoakustische Abbildungsvorrichtung nach Anspruch 5, wobei der Detektor eine Vielzahl von Empfangselementen enthält und das Verarbeiten einem Durchschnittbilden von Daten von allen Erfassungssignalen, die an der Vielzahl der Empfangselemente des Detektors erfasst werden, entspricht.

7. Fotoakustische Abbildungsvorrichtung nach Anspruch 1, ferner mit:

einer ersten flachen Platte und einer zweiten flachen Platte, die im Wesentlichen parallel zueinander angeordnet sind und konfiguriert sind, die Probe von beiden Seiten der Probe zu halten, und

einer ersten Lichtquelle, die konfiguriert ist, die erste Oberfläche mit Licht durch die erste flache Platte zu bestrahlen,

wobei der Detektor so angeordnet ist, dass er akustisch in Kontakt mit der zweiten flachen Platte steht und das erste Erfassungssignal durch Erfassen der ersten akustischen Welle ausgibt, die an der ersten Oberfläche der Probe erzeugt wird, die durch die erste flache Platte gehalten wird.

8. Fotoakustische Abbildungsvorrichtung nach Anspruch 7, wobei die Signalverarbeitungseinheit konfiguriert ist, die durchschnittliche Schallgeschwindigkeit in dem inneren Abschnitt der Probe aus einem Abstand zwischen dem Detektor und der ersten Oberfläche der Probe, die an der ersten flachen Platte gehalten wird, und aus einer ersten Zeit von dann, wenn die Probe mit dem Licht bestrahlt wird, bis dann, wenn die erste akustische Welle erfasst wird, zu berechnen.

9. Fotoakustische Abbildungsvorrichtung nach Anspruch 8, wobei die Signalverarbeitungseinheit konfiguriert ist, eine zweite Zeit, die für die erste akustische Welle benötigt wird, um durch den inneren Abschnitt der Probe zu propagieren, durch Abziehen einer Zeit, die es braucht, damit die erste akustische Welle durch die zweite flache Platte propagiert, von der ersten Zeit zu berechnen, und die durchschnittliche Schallgeschwindigkeit in dem inneren Abschnitt der Probe durch Teilen eines Abstands zwischen der ersten flachen Platte und der zweiten flachen Platte durch die zweite Zeit zu berechnen.

10. Fotoakustische Abbildungsvorrichtung nach Anspruch 7, wobei die Signalverarbeitungseinheit konfiguriert ist, die durchschnittliche Schallgeschwindigkeit in dem inneren Abschnitt der Probe basierend auf einer Differenz zwischen einer Erfassungszeit der ersten akustischen Welle und einer Erfassungszeit einer zweiten akustischen Welle und einem Abstand zwischen der Oberfläche der Probe, an der die erste akustische Welle erzeugt wird, und der Oberfläche der Probe, an der die zweite akustische Welle erzeugt wird, zu berechnen, wobei die ersten und zweiten akustischen Wellen von den Oberflächen der Probe, die durch die ersten und zweiten flachen Platten gehalten wird, erzeugt werden, und die Erfassungszeiten durch den Detektor erfasst werden.

11. Fotoakustische Abbildungsvorrichtung nach einem der Ansprüche 7 bis 10, ferner mit:

einer zweiten Lichtquelle, die verschieden von der ersten Lichtquelle ist,

wobei die zweite Lichtquelle konfiguriert ist, die zweite Oberfläche mit Licht von der zweiten Lichtquelle durch die zweite flache Platte synchron mit der ersten Lichtquelle zu bestrahlen.

12. Fotoakustisches Abbildungsverfahren, bei dem Erfassungssignale durch Erfassen von akustischen Wellen, die an Oberflächen und an einem inneren Abschnitt einer Probe durch Bestrahlen der Probe mit Licht erzeugt werden, ausgegeben werden und Bilddaten unter Verwendung des Erfassungssignals erzeugt werden, wobei das Verfahren die Schritte aufweist:

Berechnen einer durchschnittlichen Schallgeschwindigkeit in dem inneren Abschnitt der Probe unter Verwendung eines ersten Erfassungssignals einer ersten akustischen Welle, die an einer ersten Oberfläche der Probe erzeugt wird und durch den inneren Abschnitt der Probe propagiert, und

Erzeugen der Bilddaten unter Verwendung der durchschnittlichen Schallgeschwindigkeit und der Erfassungssignale.

13. Programm, das, wenn es auf einem Computer ausgeführt wird, veranlasst, dass der Computer die "berechnungs-" und "Erzeugungs-" Schritte des fotoakustischen Abbildungsverfahrens nach Anspruch 12 durchführt.

**Revendications**

1. Appareil d'imagerie photoacoustique, comprenant :

un détecteur (17) configuré pour délivrer en sortie des signaux de détection en détectant des ondes acoustiques générées au niveau des surfaces et d'une partie interne d'un échantillon par irradiation de l'échantillon avec de la lumière ; et

une unité de traitement de signal (20) configurée pour générer des données d'image en utilisant les signaux de détection,

dans lequel le détecteur est configuré pour délivrer en sortie un premier signal de détection en détectant une première onde acoustique qui est générée au niveau d'une première surface de l'échantillon et qui s'est propagée à travers la partie interne de l'échantillon,

dans lequel l'unité de traitement de signal est configurée pour calculer une vitesse moyenne du son dans la partie interne de l'échantillon en utilisant le premier signal de détection et pour générer les données d'image en utilisant la vitesse moyenne du son et les signaux de détection.

2. Appareil d'imagerie photoacoustique selon la revendication 1, dans lequel l'unité de traitement de signal est configurée pour calculer la vitesse moyenne du son dans la partie interne de l'échantillon à partir d'une distance entre la première surface de l'échantillon et le détecteur et à partir d'un temps allant du moment où l'échantillon est irradié avec la lumière jusqu'au moment où la première onde acoustique est détectée.

3. Appareil d'imagerie photoacoustique selon la revendication 1, dans lequel le détecteur est configuré pour délivrer en sortie un deuxième signal de détection en détectant une deuxième onde acoustique générée au niveau d'une deuxième surface de l'échantillon différente de la première surface de l'échantillon parmi les surfaces de l'échantillon, dans lequel l'unité de traitement de signal est configurée pour calculer la vitesse moyenne du son dans la partie interne de l'échantillon à partir du premier signal de détection et du deuxième signal de détection.

4. Appareil d'imagerie photoacoustique selon la revendication 3, dans lequel l'unité de traitement de signal est configurée pour calculer la vitesse moyenne du son dans la partie interne de l'échantillon en divisant une distance entre la première surface de l'échantillon et la deuxième surface de l'échantillon par une différence entre un temps de détection de la première onde acoustique et un temps de détection de la deuxième onde acoustique.

5. Appareil d'imagerie photoacoustique selon l'une quelconque des revendications 1 à 4, dans lequel l'unité de traitement de signal est configurée, sur la base de différences des caractéristiques de propagation entre les ondes acoustiques générées au niveau des surfaces de l'échantillon et l'onde acoustique générée au niveau de la partie interne de l'échantillon, pour effectuer un traitement d'intensification des signaux de détection obtenus à partir des ondes acoustiques générées au niveau des surfaces de l'échantillon, pour spécifier les signaux de détection obtenus à partir des ondes acoustiques générées au niveau des surfaces de l'échantillon, et pour calculer la vitesse moyenne du son dans la partie interne de l'échantillon à partir des signaux de détection générés au niveau des surfaces de l'échantillon.

6. Appareil d'imagerie photoacoustique selon la revendication 5, dans lequel le détecteur comporte une pluralité d'éléments de réception, et le traitement correspond à un calcul de moyenne des éléments de données de tous les signaux de détection détectés au niveau de la pluralité d'éléments de réception du détecteur.

7. Appareil d'imagerie photoacoustique selon la revendication 1, comprenant en outre :

une première plaque plate et une deuxième plaque plate disposées sensiblement parallèles entre elles et configurées pour maintenir l'échantillon des deux côtés de l'échantillon, et une première source de lumière configurée pour irradier la première surface avec de la lumière à travers la première plaque plate, dans lequel le détecteur est disposé de manière à entrer en contact acoustique avec la deuxième plaque plate et à délivrer en sortie le premier signal de détection en détectant la première onde acoustique générée au niveau de la première surface de l'échantillon maintenu par la première plaque plate.

8. Appareil d'imagerie photoacoustique selon la revendication 7, dans lequel l'unité de traitement de signal est configurée pour calculer la vitesse moyenne du son dans la partie interne de l'échantillon à partir d'une distance entre le détecteur et la première surface de l'échantillon maintenu sur la première plaque plate et à partir d'un premier temps allant du moment où l'échantillon est irradié avec la lumière jusqu'au moment où la première onde acoustique est détectée.

9. Appareil d'imagerie photoacoustique selon la revendication 8, dans lequel l'unité de traitement de signal est configurée pour calculer un deuxième temps requis pour la propagation de la première onde acoustique à travers la partie interne de l'échantillon en soustrayant un temps nécessaire à la propagation de la première onde acoustique à travers la deuxième plaque plate d'un premier temps, et pour calculer la vitesse moyenne du son dans la partie interne de l'échantillon en divisant une distance entre la première plaque plate et la deuxième plaque plate par le deuxième temps.

10. Appareil d'imagerie photoacoustique selon la revendication 7, dans lequel l'unité de traitement de signal est configurée pour calculer la vitesse moyenne du son dans la partie interne de l'échantillon sur la base d'une différence entre un temps de détection de la première onde acoustique et un temps de détection d'une deuxième onde acoustique et d'une distance entre la surface de l'échantillon où la première onde acoustique est générée et la surface de l'échantillon où la deuxième onde acoustique est générée, les première et deuxième ondes acoustiques étant générées à partir des surfaces de l'échantillon maintenu par les première et deuxième plaques plates, les temps de détection étant détectés par le détecteur.

11. Appareil d'imagerie photoacoustique selon l'une quelconque des revendications 7 à 10, comprenant en outre :

une deuxième source de lumière différente de la première source de lumière, dans lequel la deuxième source de lumière est configurée pour irradier la deuxième surface avec de la lumière provenant de la deuxième source de lumière à travers la deuxième plaque plate en synchronisme avec la première source de lumière.

12. Procédé d'imagerie photoacoustique, dans lequel des signaux de détection sont délivrés en sortie en détectant des ondes acoustiques générées au niveau des surfaces et d'une partie interne d'un échantillon par irradiation de l'échantillon avec de la lumière, et des données d'image sont générées en utilisant le signal de détection, le procédé comprenant les étapes consistant :

à calculer une vitesse moyenne du son dans la partie interne de l'échantillon en utilisant un premier signal de détection d'une première onde acoustique générée au niveau d'une première surface de l'échantillon et se propageant à travers la partie interne de l'échantillon, et à générer les données d'image en utilisant la vitesse moyenne du son et les signaux de détection.

13. Programme qui, lorsqu'il est exécuté sur un ordinateur, amène l'ordinateur à effectuer les étapes de « calcul » et de « génération » du procédé d'imagerie photoacoustique selon la revendication 12.

[Fig. 1]

[Fig. 2]

S201　ANALYZE DETECTION SIGNAL DATA, AND CALCULATE
FIRST TIME ($t_{surface}$) LASTING FROM IRRADIATION USING PULSED LIGHT
TO DETECTION OF FIRST ACOUSTIC WAVE

S202　CALCULATE AVERAGE SOUND SPEED IN INNER PORTION OF
SAMPLE FROM FIRST TIME ($t_{surface}$) AND DISTANCE
BETWEEN SURFACE OF SAMPLE AND DETECTOR

S203　USING CALCULATED AVERAGE SOUND SPEED,
PROCESS DETECTION SIGNAL OF ACOUSTIC WAVE GENERATED
AT INNER PORTION OF SAMPLE, AND FORM IMAGE DATA
OF INNER PORTION OF SAMPLE

[Fig. 3]

[Fig. 4A]

1510 m/sec.

[Fig. 4B]

1540 m/sec.

[Fig. 5A]

[Fig. 5B]

[Fig. 5C]

[Fig. 6]

**EP 2 533 697 B1**

## REFERENCES CITED IN THE DESCRIPTION

**Patent documents cited in the description**

- EP 1935346 A